# NEW EUROPEAN PATENT SPECIFICATION

(11) **EP 1 109 768 B2**
(45) Date of publication and mention of the opposition decision: **18.04.2007**
(45) Mention of the grant of the patent: 15.01.2003
(21) Application number: 99932283.7
(22) Date of filing: 06.07.1999
(51) Int. Cl.: C07C 39/06, C07C 37/84

(54) **PURIFICATION OF 2, 4, 6 - TRI - (TERT BUTYL) PHENOL BY MELT CRYSTALLIZATION**
VERFAHREN ZUR REINIGUNG VON 2, 4, 6 - TRI - (TERT BUTYL) PHENOL DURCH SCHMELZKRISTALLISIERUNG
PURIFICATION DE 2, 4, 6 - TRI - (TERT BUTYL) PHENOL AU MOYEN D'UNE CRISTALLISATION PAR FUSION

(30) Priority: 03.09.1998 US 146898
(43) Date of publication of application: 27.06.2001
(73) Proprietor: Chemtura Corporation, Middlebury, CT 06749 (US)
(72) Inventor: MAHOOD, james, Alan, Morgantown, WV 26505 (US)
(74) Representative: Wibbelmann, Jobst
(86) International application number: PCT/US1999/015241
(87) International publication number: WO 2000/014043

(56) References cited:
- EP-A- 0 146 170
- EP-A1- 0 511 947
- EP-B1- 0 143 472
- DE-A1- 1 813 840
- DE-A1- 3 717 282
- DE-B1- 2 531 774
- GB-A- 655 124
- JP-A1- 2 229 125
- ULLMAN'S ENCYCLOPEDIA, 1974, BAND 2, S. 683
- CHEMICAL ENGINEERING PROGRESS, MARCH 1992
- ULLMAN'S ENCYCLOPEDIA, 1972, BAND 18, S. 191 - 195

## Description

This invention relates to a novel method for recovering substantially pure 2,4,6-tri-(tert-butyl)phenol. More particularly, the invention is directed to the recovery of 2,4,6-tri-(tert-butyl)phenol by subjecting a mixture comprising alkylated phenols to a method that does not require, among other things, adduct crystallizations and the generation of reaction recycle streams.

Short chain alkylphenols are commercially important end products and process intermediates. As end products they are used as antioxidants, stabilizers, and additives, for example, as radical trapping agents in plastics, elastomers, synthetic fibers, fuels, lubricants, and foods.

It is known to prepare alkylphenols via the alkylation of phenolic compounds with such alkylating agents as iso-butylene and tertiary-butyl alcohol, and a variety of alkylation catalysts are known to promote such a reaction. For example, U.S. Patent Nos. 3,959,394; 3,439,048; 3,426,358; 3,426,082; 3,409,678; 3,382,283; 3,367,981; 3,265,742; 3,185,737; 3,133,974; 3,082,258 and 3,071,595, all disclose alkylation of phenols over a variety of catalyst such as Friedel-Crafts catalysts (AlCl₃, HF, BF₃, etc.), zinc halides, alumina, aluminum phenoxide, alkane sulfonic acids, ion exchange resins, and the like. Production of these compounds by these conventional methods is, however, somewhat problematic in that the reaction yields a broad spectrum of products. Isolation or enrichment of specific positional isomers is both difficult and expensive. Crude 2,4,6-tri-(tert-butyl)phenol is often available as a bottoms product from the manufacture of 2,4-di-(tert-butyl)phenol and 2,6-di-(tert-butyl)phenol, both of which can be conveniently isolated by standard distillation techniques. Unfortunately, the remaining 2,4,6-tri-(tert-butyl)phenol has a high melting point that leads to column freeze-up problems when distilled. Moreover, the boiling point difference between 2,4,6-tri-(tert-butyl)phenol and 2,5-di-(tert-butyl)phenol, a common co-impurity, is so small that separation is extremely challenging and expensive. Solvent crystallization is not desirable due to the environmental hazards associated with handling solvents as well as the small solubility differences between 2,4,6-tri-(tert-butyl)phenol and 2,5-di-(tert-butyl)phenol.

It is apparent that a need continues to exist for methods to purify alkylated phenols, especially 2,4,6-tri-(tert-butyl)phenol, from mixtures containing alkylated phenols.

EP-A-0146170 discloses a process in which a raw 2,6-xylenol product is purified by melting it, then slowly and uniformly cooling it until at least 60 to 100% is crystallized, with the greater concentration of the impurities being present nearer the surface of the resulting crystals, gradually heating the crystalline solid mass to an elevated temperature no higher than by 0.1 to 5°C below the solidification temperature of 2,6-xylenol to cause only surface melting of the crystals, thus yielding a molten liquid phase and a solid crystalline phase, the solid phase containing 2,6-xylenol in greater purity than the molten phase.

GB-A-655124 discloses higher poly-tert-butyl phenol or cresols are separated from lower tert-butylated phenol or cresols in the crude reaction mixture resulting from the alkylation of phenol or a cresol with isobutylene or a butyl halide in the presence of an alkylation catalyst by a process in which the crude mixture is cooled to a temperature at which the higher poly-tert-butyl phenol or cresols precipitate out, and these are then separated, and washed to remove catalyst. The process may be operated continuously. In the examples phenol is alkylated with a C₄ refinery cut containing isobutylene, in the presence of sulphuric acid, and the tri-tert-butylphenol separated at 40°C., and p-cresol is similarly alkylated with the C₄ refinery cut and di-tert-butyl-p-cresol separated at room temperature.

The instant invention is directed to a method for recovering 2,4,6-tri-(tert-butyl)phenol which is at least about 99% pure in the absence of adduct crystallizations and without the generation of reaction recycle streams, said method comprising the steps of:
a) vacuum distilling a mixture comprising said 2,4,6-tri-(tert-butyl)phenol to produce a distillate and a residue, wherein the residue comprises 2,4,6-tri-(tert-butyl)phenol and at least one member of the group consisting of 2,4-di-(tert-butyl)phenol, 2,5-di-(tert-butyl)phenol, 2,6-di-(tert-butyl)phenol, 2,6-di-(tert-butyl)-4-methylphenol, 2-(tert-butyl)phenol, and 4-(tert-butyl)phenol, and
b) subjecting the residue to melt crystallization to obtain 2,4,6-tri-(tert-butyl)phenol which is at least about 99% pure.

It has been unexpectedly discovered that substantially pure 2,4,6-tri-(tert-butyl)phenol may be recovered from mixtures comprising the same in the absence of adduct crystallizations and without the generation of reaction recycle streams. Reaction recycle stream is defined herein to mean a stream comprising impurities fed back to further react in the process which is the source of the mixture comprising the desired alkylated phenol, 2,4,6-tri-(tert-butyl)phenol.

For example, the mixtures employed in this invention may be the reaction mixtures generated from processes to manufacture substituted alkylphenols made by reacting phenol or 2-tert-butylphenol with 2-methyl-1-propene in the liquid phase, optionally in the presence of inert diluents and/or an excess of the alkene, wherein the reaction is carried out in the presence of a catalyst. Such processes are known in the art as illustrated by crystallization step. Said mixtures comprising the alkylated phenol may therefore be solids, melts, or solutions.

The mixtures in this invention subject to the melt crystallization step comprise 2,4,6-tri-(tert-butyl)phenol, 2,4-di-(tert-butyl)phenol, 2,6-di-(tert-butyl)-4-methylphenol, 2,5-di-(tert-butyl)phenol, 2,6-di-(tert-butyl)phenol, 2-(tert-butyl)phenol, and/or 4-(tert-butyl)phenol. The ratios of the various components may also vary significantly, however, it is preferable for the mixture to have already undergone purification to recover the intended alkylphenols thus leaving the fewer alkylated phenols in the mixture for recovery. In one embodiment, it is desired to remove the alkylphenols intended by the reaction to leave the alkylated phenol intended to be recovered by melt crystallization, 2,4,6-tri-(tert-butyl)phenol, in increased proportions for recovery as compared to a crude alkylation reaction mixture.

Accordingly, when conducting the instant invention, the mixtures comprising the alkylated phenols are preferably first fed to a set-up/apparatus that is capable of enabling the mixture comprising the alkylated phenols is be distilled to produce a residue enriched in the alkylated phenol intended for recovery by melt crystallization 2,4,6-tri-(tert-butyl)phenol. Such a setup/apparatus is not limited and it is often one that comprises a vacuum source, heat source, distillation flask, and a condenser.

There is essentially no limitation with respect to the temperature at which the distillation takes place other than that the temperature is not one which causes disintegration of the alkylated phenols and is not greater than the boiling point of the alkylated phenol intended to be recovered by melt crystallization, 2,4,6-tri-(tert-butyl)phenol, at the pressure the distillation takes place. The pressure at which the distillation takes place is often no more than about 40.0 kPa (300 torr) preferably no more than about 13.3 kPa (100 torr) and most preferably, no more than about 1.33 kPa (10 torr).

Subsequent to the distillation step, the resulting residue comprising the alkylated phenol intended to be recovered, 2,4,6-tri-(tert-butyl)phenol, is melt crystallized. It is preferred that substantial quantities of the lower boiling alkylated phenols and any phenol that may have been used as a starting material be removed prior to melt crystallization. Any apparatus capable of removing impurities from a melt of the residue may be employed. Often, the melt crystallization is achieved via a zone melting or zone refining apparatus. Such an apparatus often comprises a means for freezing and melting the residue. The melting and freezing temperatures at which the residue is subjected to are those which allow for the substantially pure alkylated phenol, 2,4,6-tri-(tert-butyl)phenol, to crystallize and impurities to collect in the resulting molten phase. An illustrative example of such an apparatus may be found in Modern Methods of Chemical Analysis(1968), pages 15-16.

The crude 2,4,6-tri-(tert-butyl)phenol, used for the melt crystallization contains 2,4,6-tri-(tert-butyl)phenol, and side reaction products such as, 2,4-di-(tert-butyl)phenol, 2,5-di-(tert-butyl)phenol, 2,6-di-(tert-butyl)phenol, 2,6-di-(tert-butyl)-methylphenol, 2-(tert-butyl)phenol, and 4-(tert-butyl)phenol. The composition in its melt form is subjected to fractional melt crystallization, usually multistage. The temperature is lowered gradually until the temperature is somewhat below the melting point of the desired substance, 2,4,6-tri-(tert-butyl)phenol, melting point of about 132°C. In some cases, the composition may have to be heated above the melting temperature of the desired alkylated phenol and then brought down below its freezing point. Clearly this particular procedure is advantageous in separating the component crystallizes onto the surface of the vessel holding the melt composition. The theory of the fractional melt crystallization is that the desired component preferentially is crystallized out from the melt while the undesired impurities remain in their liquid state or are entrapped in the crystalline medium to a limited extent. In a multiple stage fractional melt crystallization, the crystalline desired component's purity is upgraded in each successive stage, through the phases of crystallization, partial melting (sweating), and total melting.

Preferred apparatus to carry out the fractional melt crystallization is referred to as the "Sulzer" melt crystallization apparatus. This is a falling film dynamic crystallizer, which is obtained from Sulzer Canada, Inc., a subsidiary of Sulzer Brothers, Ltd., Switzerland. An example of such an apparatus obtained from Sulzer Canada and general multistage fractional melt crystallization procedure is disclosed in detail in U.S. Pat. No. RE 32,241 (3,621,664) issued to K. Saxer. This type of crystallizer is substantially different from a single stage, static state crystallizer, for example as shown in Konecny Czech patent publication 246681 wherein bisphenol-A is purified in a static crystallizer. The continued detailed explanation of the invention is now disclosed with respect to the Sulzer-type of apparatus.

A crude 2,4,6-tri-(tert-butyl)phenol mixture is used containing products of the various side reactions. The major impurity component is usually 2,5-di-(tert-butyl)phenol. The crude 2,4,6-tri-(tert-butyl)phenol stream is fed to a fractional melt crystallization apparatus, preferably utilizing falling films. This apparatus generally comprises a group of tubes with central distribution system to each tube, a liquid circulation system for both the heat exchange medium and the 2,4,6-tri-(tert-butyl)phenol containing melts, a circulation pump, a collecting tank at the bottom of the tubes, a feed tank for each stage which also functions as a holding tank for residue from crystallization phase (mother liquor) and sweat liquor, the process being conducted in multiple stages with three phases in each stage-a crystallization phase, a sweat phase and a melt phase. Each successive stage produces a purer form of the 2,4,6-tri-(tert-butyl)phenol, the number of stages being sufficient to obtain the desired final purity in high yield and thereby finishing one complete cycle.

The surface of the tubes is a medium on which the 2,4,6-tri-(tert-butyl)phenol can readily crystallize usually metal. The heat transfer medium can be inside the tube or outside, preferably on the outside of the tube. The composition which is to be crystallized is preferably inside the tube. The composition from which the desired component is to be recovered can substantially fill the tube. Similarly the heat transfer medium can substantially fill the space outside the tube. However, it is preferred that both the composition to be purified and the heat transfer medium contact the inner surface and outer surface of the tube, respectively, as a film which falls from the top of the tube to the collecting reservoir. As the crude composition falls down the tube, the temperature of the heat transfer medium is lowered until the temperature of the wall on which desired compound, 2,4,6-tri-(tert-butyl)phenol, is crystallized is below the melting point of the 2,4,6-tri-(tert-butyl)phenol, for example, about 2°C to 5°C below. At this point, crystals of 2,4,6-tri-(tert-butyl)phenol will crystallize out on the surface of the tube. As the crystals build on the surface of the tube, the temperature should be gradually lowered to compensate for the thickness of the crystalline surface, and the declining freezing point of the melt. This completes the crystallization phase. The liquid which is not crystallized (i.e. the residue) collects in the sump tank. At that point it can be shifted to an intermediate holding tank.

At this point the "sweat" phase can begin. In this phase of the stage, the temperature of the heat transfer medium is gradually raised. This allows some of the crystallized 2,4,6-tri-(tert-butyl)phenol on the tube surface to remelt, thus carrying away some of the entrapped impurities as well as adsorbed mother liquor. The sweat "liquor" is collected in the sump tank and is then passed to an intermediate holding tank for the sweat liquor. Finally, the temperature is raised significantly and the remaining crystalline 2,4,6-tri-(tert-butyl)phenol is melted. This completes one stage of a multi stage cycle. The purified melted 2,4,6-tri-(tert-butyl)phenol is now combined with the sweat liquor from a higher stage of a previous purification cycle and falls down the inside portion of the tube as a film. This feed stock is now subjected to the same three phases of crystallization, sweating and melting. The melt residue from the crystallization phase going to its holding tank, the sweat liquor to its holding tank, and the melt of crystals being of sufficient purity to be isolated in whatever form, for example prilling or flaking. The sweat liquor and crystallization residue from a previous cycle are combined and caused to fall in a film down a tube wherein the three phases of crystallization, sweating and melting are once more carried out. In this case, the residue from the crystallization phase is passed back to the 2,4,6-tri-(tert-butyl)phenol process as recycle.

The crystal melt is combined with new crude 2/4,6-tri-(tert-butyl)phenol fed from the 2,4,6-tri-(tert-butyl)phenol process and also combined with the sweat liquor from a previous cycle and the impure residue from the previous cycle and processed in the tubes through the three phases of crystallization, sweat and melt to obtain more purified 2,4,6-tri-(tert-butyl)phenol, sweat liquor, and impure residue, the sweat liquor and impure residue being stored in holding tanks for the proper stage of a future cycle. This is a process wherein the three phases of crystallization, sweat and melt make up a single stage; depending upon the purity of the feed, multiple stages are generally used to obtain the purification desired and these multiple stages make up a single product cycle.

The following example is provided to further illustrate and facilitate the understanding of the instant invention. All products obtained may be confirmed via conventional techniques including proton and carbon-13 magnetic resonance spectroscopy, infrared spectroscopy and x-ray techniques.

The alkylations of phenol or 2-alkylphenols to prepare mixtures containing 2,4,6-tri-(tert-butyl)phenol are generally carried out using isobutene in a stirred autoclave. Temperature control is maintained by a high-powered temperature controller using a temperature sensor placed in the interior of the reactor allowed monitoring of the reaction temperature. A suitable measuring device is often employed to allow reactor level changes to be monitored. Samples may be taken for gas chromatographic analysis via a nipple on the reactor. Prior to determination of the proportions of the various components of the reaction mixture by gas chromotagraphy, the catalyst contained in the sample is typically deactivated by addition of a few drops of water.

By the method described above, for Example 1, 251 g (1.67 mol) 2-tert-butylphenol (2-TBP) was reacted at 1.5 bar and 10°C with 1.9-2.0 mol isobutene in the presence of 5.8 mol aluminum tris(2-tert-butylphenolate). In Example 2, 11.8 mmol of 2,4,6-trichlorophenol co-catalyst was present. The samples taken after a 180 minute reaction time showed high conversions to 2,6-di-tert-butylphenol (2,6-DTBP) with 2,4,6-tri-tert-butylphenol (2,4,6-TTBP) and other by-products, including 2,5-di-tert-butylphenol. Typical product distributions are as follows.

| | | | | |
|---|---|---|---|---|
| Example 2 | 0.7 | 93.7 | 4.9 | 0.7 |

A typical reaction mixture containing the alkylated phenols as shown in the above examples was distilled via vacuum distillation {pressure 0.60 kPa (4.5 torr) (bottom) 0.36 kPa (2.7 torr)(top), temperature 230°C. (bottom), 210°C. (top)} to remove first the lower boiling 2-TBP followed by the desired 2,6-DTBP. The resulting residue (2,4,6-TTBP enriched) was fed to a zone refining apparatus for melt crystallization. In the melt crystallization, a melt of the alkylated phenols was cooled to about 105°C, followed by sweating to about 128°C. Gas chromatography results showing the alkylated phenol product distributions for the melt crystallization are as follows:

| | Feed | Drain | Sweat | Melt |
|---|---|---|---|---|
| 2,6-DTBP | 2.89 | 6.95 | 5.48 | 0.02 |
| 2,4-DTBP | 3.29 | 5.72 | 4.82 | 0.02 |
| 2,5-DTBP | 5.76 | 9.38 | 7.83 | 0.03 |
| 2,4,6-TTBP | 88.06 | 76.29 | 80.4 | 99.93 |

The resulting final product, 2,4,6-tri-tert-butylphenol (2,4,6-TTBP) was greater than 99.9% pure, indicating that a substantially pure product could be obtained in the absence of adduct crystallizations and without the generation of reaction recycle streams.

## Claims

1. A method for recovering 2,4,6-tri-(tert-butyl)phenol which is at least about 99% pure in the absence of adduct crystallizations and without the generation of reaction recycle streams, said method comprising the steps of:
a) vacuum distilling a mixture comprising said 2,4,6-tri-(tert-butyl)phenol to produce a distillate and a residue, wherein the residue comprises 2,4,6-tri-(tert-butyl)phenol and at least one member of the group consisting of 2,4-di-(tert-butyl)phenol, 2,5-di-(tert-butyl)phenol, 2,6-di-(tert-butyl)phenol, 2,6-di-(tert-butyl)-4-methylphenol, 2-(tert-butyl)phenol, and 4-(tert-butyl)phenol; and
b) subjecting the residue to melt crystallization to obtain 2,4,6-tri-(tert-butyl)phenol which is at least about 99% pure.

2. The method of claim 1, wherein said melt crystallization is accomplished via a zone melting or a zone refining apparatus.

## Patentansprüche

1. Ein Verfahren zum Gewinnen von 2,4,6-Tri-(tert-butyl)phenol in mindestens etwa 99%iger Reinheit ohne Addukt-Kristallisationen und ohne die Erzeugung von Reaktions-Rückführungsströmen, wobei das Verfahren die Stufen umfasst:
a) Vakuumdestillieren einer Mischung, umfassend das 2,4,6-Tri-(tert-butyl)phenol zur Herstellung eines Destillates und eines Restes, wobei der Rest das 2,4,6-Tri-(tert-butyl)phenol und mindestens ein Mitglied aus der Gruppe bestehend aus 2,4-Di-(ter-butyl)phenol, 2,5-Di-(tert-butyl)phenol, 2,6-Di-(tert-butyl)phenol, 2,6-Di-(tert-butyl)-4-methylphenol, 2-(tert-Butyl)phenol und 4-(tert-Butyl)phenol umfasst, und
b) Schmelzkristallisieren des Restes, um 2,4,6-Tri-(tert-butyl)phenol zu erhalten, das mindestens etwa 99% rein ist.

2. Das Verfahren nach Anspruch 1, worin die Schmelzkristallisation mittels einer Vorrichtung zum Zonenschmelzen oder zum Zonenreinigen ausgeführt wird.

## Revendications

1. Procédé destiné à récupérer du 2,4,6-tri-(tert-butyl)phénol qui est pur à au moins environ 99 % en l'absence de cristallisations d'adduit et sans génération de courants de recyclage de réaction, ledit procédé comprenant les étapes :
a) de distiller sous vide un mélange comprenant ledit 2,4,6-tri-(tert-butyl)phénol pour produire un distillat et un résidu, dans lequel le résidu comprend du 2,4,6-tri-(tert-butyl)phénol et au moins un élément du groupe consistant en le 2,4-di-(tert-butyl)phénol, le 2,5-di-(tert-butyl)phénol, le 2,6-di-(tert-butyl)phénol, le 2,6-di-(tert-butyl)-4-méthylphénol, le 2-(tert-butyl)phénol et le 4-(tert-butyl)phénol ; et
b) de soumettre le résidu à une cristallisation à l'état fondu pour obtenir le 2,4,6-tri-(tert-butyl)phénol qui est pur à au moins environ 99 %.

2. Procédé selon la revendication 1, dans lequel ladite cristallisation à l'état fondu est réalisée par l'intermédiaire d'un appareil de fusion de zone ou de raffinage de zone.
